(19) Europäisches Patentamt
European Patent Office
Office européen des brevets

(11) **EP 1 077 950 B1**

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(21) Anmeldenummer: **99920662.6**

(22) Anmeldetag: **14.04.1999**

(51) Int Cl.[7]: **C07D 239/26**, C07D 239/36, C07D 251/22, C07D 253/06, A61K 31/505, A61K 31/53

(86) Internationale Anmeldenummer:
**PCT/EP1999/002497**

(87) Internationale Veröffentlichungsnummer:
**WO 1999/054311 (28.10.1999 Gazette 1999/43)**

(54) **NEUE DIPHENYL-SUBSTITUIERTE 6-RING HETEROCYCLEN, VERFAHREN ZU IHRER HERSTELLUNG SOWIE DEREN VERWENDUNG ALS ARZNEIMITTEL**

NOVEL DIPHENYL-SUBSTITUTED 6-RING-HETEROCYCLES, METHODS FOR PRODUCING THEM AND THEIR USE AS MEDICAMENTS

NOUVEAUX HETEROCYCLES A 6 NOYAUX A SUBSTITUTION DIPHENYLE, LEUR PROCEDE DE PREPARATION ET LEUR UTILISATION COMME MEDICAMENTS

(84) Benannte Vertragsstaaten:
**AT BE CH CY DE DK ES FI FR GB GR IE IT LI LU MC NL PT SE**

(30) Priorität: **17.04.1998 IT MI980819**

(43) Veröffentlichungstag der Anmeldung:
**28.02.2001 Patentblatt 2001/09**

(73) Patentinhaber:
- **Boehringer Ingelheim Pharma GmbH & Co.KG 55218 Ingelheim am Rhein (DE)**
- **BOEHRINGER INGELHEIM ITALIA S.p.A. 20139 Milano (IT)**

(72) Erfinder:
- **BRENNER, Michael D-55411 Bingen am Rhein (DE)**
- **PALLUK, Rainer D-55411 Bingen (DE)**
- **WIENRICH, Marion D-64331 Weiterstadt (DE)**
- **WEISER, Thomas D-55268 Nieder-Olm (DE)**
- **CEREDA, Enzo I-15067 Novi Ligure (IT)**
- **BIGNOTTI, Maura I-20135 Milano (IT)**
- **PELLEGRINI, Carlo Maria I-26841 Casalpusterlengo (IT)**
- **SCHIAVI, Giovanni, Battista I-46041 Asola (IT)**
- **CESANA, Raffaele I-20125 Milano (IT)**

(74) Vertreter: **Laudien, Dieter, Dr.**
**Boehringer Ingelheim GmbH**
**Abteilung Patente**
**55216 Ingelheim (DE)**

(56) Entgegenhaltungen:

| | |
|---|---|
| **EP-A- 0 086 411** | **EP-A- 0 635 500** |
| **EP-A- 0 713 703** | **EP-A- 0 781 766** |
| **WO-A-98/17652** | **US-A- 3 821 221** |
| **US-A- 3 969 355** | **US-A- 5 521 189** |

- **KUNO ET AL.: "Studies on cerebral protective..." CHEM.PHARM.BULL., Bd. 40, Nr. 6, 1992, Seiten 1452-1461, XP002113911**
- **KUMAR A ET AL: "Anti-Pneumocystis carinii pneumonia activity of dicationic 2,4 -diarylpyrimidines" EUROPEAN JOURNAL OF MEDICINAL CHEMISTRY.CHIMICA THERAPEUTICA, Bd. 31, Nr. 10, 1. Januar 1996 (1996-01-01), Seite 767-773 XP004070190 ISSN: 0223-5234**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Diphenyl-substituierte 6-Ring Heterocyclen, Verfahren zu ihrer Herstellung sowie deren Verwendung als Arzneimittel.

Die neuen Verbindungen besitzen die Struktur der allgemeinen Formel (I)

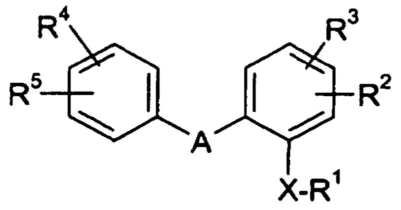

(I)

worin

A ein 6-gliedriger gesättigter oder ungesättigter Heterocyclus, der als Heteroatome 1, 2 oder 3 Stickstoffatome enthalten kann und gegebenenfalls ein- oder mehrfach durch =O oder $C_1$-$C_4$-Alkyl substituiert ist;

X Sauerstoff;

$R^1$ $C_1$-$C_4$-Alkyl, das durch $-NR^6R^7$ oder durch ein N-Oxid der Formel $-NOR^6R^7$ substituiert ist;

$R^2$ und $R^3$ Wasserstoff;

$R^4$ und $R^5$ Wasserstoff;

$R^6$ Wasserstoff oder $C_1$-$C_4$-Alkyl;

$R^7$ Wasserstoff oder $C_1$-$C_4$-Alkyl, oder

$R^6$ und $R^7$ bilden zusammen mit dem Stickstoffatom einen gesättigten oder ungesättigten Heterocyclus ausgewählt aus der Gruppe Pyrrol, Pyrrolin, Pyrrolidin, Piperidin, Piperazin, Morpholin, Thiomorpholin, Imidazol, Imidazolin, Imidazolidin, Pyrazol, Pyrazolin, Pyrazolidin, wobei die genannten Heterocyclen gegebenenfalls durch Methyl, Ethyl, Propyl oder Benzyl substituiert sein können, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderer Bedeutung sind ferner Verbindungen der allgemeinen Formel (I), worin

A ein 6-gliedriger Heterocyclus, ausgewählt aus der Gruppe Pyridin, Pyridazin, Pyrimidin, Pyrazin oder Triazin, der gegebenenfalls durch =O oder $C_1$-$C_4$-Alkyl substituiert sein kann;

X Sauerstoff;

$R^1$ Methyl, Ethyl oder Propyl, die jeweils durch $-NR^6R^7$ oder durch ein N-Oxid der Formel $-NOR^6R^7$ substituiert sind;

$R^2$ und $R^3$ Wasserstoff;

$R^4$ und $R^5$ Wasserstoff;

$R^6$ Wasserstoff, Methyl, Ethyl oder Propyl;

$R^7$ Wasserstoff, Methyl, Ethyl oder Propyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von Bedeutung sind ferner Verbindungen der allgemeinen Formel (I), worin die Gruppe

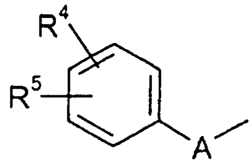

für einen der folgenden Reste stehen kann

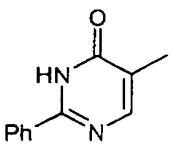 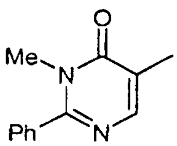 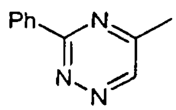 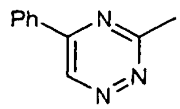

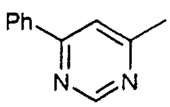 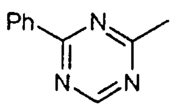 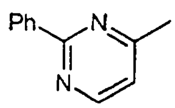 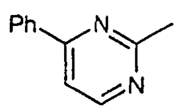 ;

X Sauerstoff;

$R^1$ Ethyl oder Propyl, die jeweils durch $-NR^6R^7$ substituiert sind;

$R^2$ und $R^3$ Wasserstoff;

$R^6$ Methyl, Ethyl oder Propyl;

$R^7$ Methyl, Ethyl oder Propyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Von besonderer Bedeutung sind ferner Verbindungen der allgemeinen Formel (I), worin die Gruppe

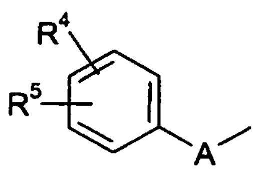

für einen der folgenden Reste stehen kann

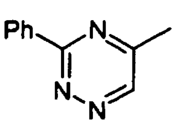 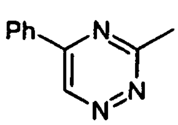 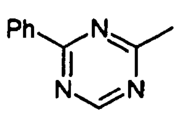 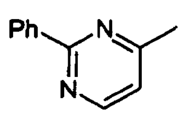 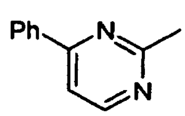 ;

X Sauerstoff;

$R^1$ $-CH_2-CH_2-NR^6R^7$;

$R^2$ und $R^3$ Wasserstoff;

$R^6$ Methyl;

$R^7$ Methyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Erfindungsgemäß von besonderer Bedeutung sind die Verbindungen 5-{2-[2(N,N-Dimethylamino)ethyl]oxy-phenyl}-3-phenyl-1,2,4-triazin 3-{2-[2(N,N-Dimethylamino)ethyl]oxy-phenyl}-5-phenyl-1,2,4-triazin 2-{2-[2(N,N-Dimethylamino)ethyl]oxy-phenyl}-4-phenyl-1,3,5-triazin und 4-{2-[2(N,N-Dimethylamino)ethyl]oxy-phenyl}-2-phenyl-pyrimidin.

Als Alkylgruppen (auch soweit sie Bestandteil anderer Reste sind, beispielsweise Alkylenbrücken) werden, soweit nicht anders angegeben, verzweigte und unverzweigte Alkylgruppen mit 1 - 4 Kohlenstoffatomen bezeichnet, beispielsweise werden genannt: Methyl, Ethyl, n-Propyl, iso-Propyl, Butyl, iso-Butyl, sec. Butyl, tert.-Butyl, Pentyl, iso-Pentyl, Hexyl, Heptyl und Octyl. Zur Bezeichnung der Gruppen Methyl, Ethyl, Butyl oder auch tert.-Butyl werden auch die Abkürzungen Me, Et, Bu oder tBu verwendet.

Als Beispiele für N-verknüpfte cyclische Reste der allgemeinen Formel $NR^6R^7$ seien genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen durch Alkyl mit 1 bis 4 Kohlenstoffatomen, bevorzugt Methyl substituiert sein können.

"=O" bedeutet ein über eine Doppelbindung verknüpftes Sauerstoffatom.

Die vorliegende Erfindung beschreibt Verbindungen, die überraschenderweise eine hohe Affinität zu folgendem Rezeptortyp aufweist: "$Na^+$ Kanal site 2" Bindungsstelle. Darüber hinaus zeigen diese Verbindungen antagonistische Aktivität am AMPA-Rezeptor. Aufgrund dieser Befunde können die erfindungsgemäßen Verbindungen bei neurodegenerativen Erkrankungen sowie Gehirnischämie verschiedener Genese eingesetzt werden.

Die erfindungsgemäßen Verbindungen können in Anlehnung an bekannte Analogieverfahren dargestellt werden. So können beispielsweise Verbindungen der allgemeinen Formel (II)

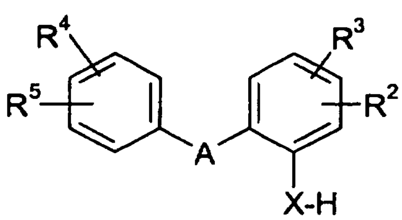

(II),

worin A, X und die Reste $R^2$, $R^3$, $R^4$ und $R^5$ die oben genannte Bedeutung aufweisen, unter basischen Bedingungen mit Elektrophilen der allgemeinen Formel

$$L-R^1,$$

wobei L eine Abgangsgruppe wie beispielsweise Chlor, Brom, Iod, Methansulfonyl, Trifluormethansulfonyl oder p-Toluolsulfonyl darstellt und $R^1$ die oben angegebene Bedeutung aufweist, unter Alkylierung zu den Verbindungen der allgemeinen Formel (I) umgesetzt werden.

Die folgenden Synthesevorschriften dienen der näheren Erläuterung der zur Darstellung der erfindungsgemäßen Verbindungen anwendbaren Analogieverfahren, ohne die Erfindung jedoch auf deren Gegenstand zu beschränken.

**Referenzbeispiel 1:** 4-(2-Hydroxyphenyl)-6-phenyl-pyrimidin

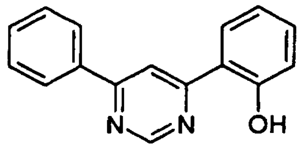

Eine Lösung von 1-(2-hydroxy phenyl)-3-phenyl-1,3-propandion (4 g) in Formamid (40 ml) wurde bei 170°C unter Rühren für 3 Stunden erhitzt. Nach Abkühlen wurde die Lösung in Wasser gegeben und mehrmals mit Diethylether extrahiert. Die organische Phase wurde getrocknet, bis zur Trockene eingedampft und der erhaltene Rückstand durch Flash-Chromatographie gereinigt (Laufmittel Cyclohexan-Ethylacetat 80-20).
Ausbeute: 1.1 g; Schmp. 170-172°C dec. (als Hydrochlorid)

**Beispiel 1:** 4-{2-[2(N,N-Dimethylamino)ethyl]oxy-phenyl}-6-phenyl-pyrimidin

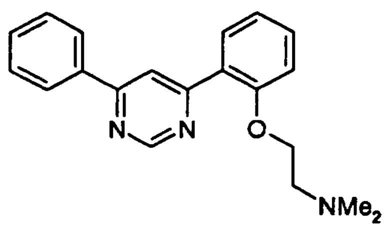

Eine Lösung von 4-(2-Hydroxyphenyl)-6-phenyl-pyrimidin (0.5 g, Referenzbeispiel 1) und NaH (0.12 g, 80% in Öl) in wasserfreiem DMF (5 ml) wurde 20 Minuten bei Raumtemperatur gerührt. Dann wurde 2-Dimethylaminoethylchloride-hydrochlorid (0.29 g) zugesetzt und die Lösung unter Rühren für 6 Stunden auf 100°C erhitzt. Nach Abkühlen wurde die Lösung in Wasser gegeben und das abgetrennte Öl mit Ethylacetat extrahiert. Nach Trocknen wurde die organische Phase bis zur Trockene eingedampft, um 500mg der Titelverbindung als Rohprodukt zu liefern, welches als Hydrochlorid gereinigt wird. Dieses wurde durch Lösen der freien Base in Aceton, Zugabe einer etherischen Salzsäurelösung und Abfiltrieren des kristallinen Salzes erhalten. Ausbeute: 0.4 g; Schmp. 135-140°C dec. (als Hydrochloride aus Aceton)

**Referenzbeispiel 2:** 4-(2-Hydroxyphenyl)-2-phenyl-pyrimidin

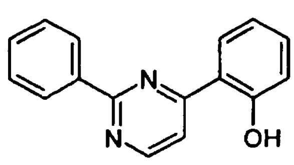

Eine Lösung von 3-Bromo-chroman-4-one (2.5 g) und Benzanüdine-hydrochlorid (1.7 g) in wasserfreiem EtOH (25 ml) wurde für 6 Stunden in Gegenwart von DBU (1.7 g) zum Rückfluß erhitzt. Nach Abkühlen wurde die Reaktionsmischung bis zur Trockene eingedampft und der verblieben Rückstand in einer Mischung aus Ethylacetat und verdünnter wässriger Salzsäure aufgenommen. Die organische Phase wurde mit verdünnter wässriger $Na_2CO_3$ Lösung und Wasser gewaschen, getrocknet und bis zur Trockene zum Rohprodukt der Titelverbindung eingedampft. Dieses wurde über das Hydrochlorid gereinigt, welches durch Lösen der freien Base in Aceton und Zugabe von etherischer Salzsäurelösung erhalten wurde.
Ausbeute: 0.85 g; Schmp. 195-198°C dec,( als Hydrochloride aus Aceton).

**Beispiel 2:** 4-{2-[2(N,N-Dimethylamino)ethyl]oxy-phenyl}-2-phenyl-pyrimidin

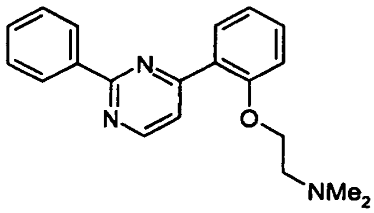

Die Darstellung erfolgte ausgehend von 4-(2-Hydroxyphenyl)-2-phenyl-pyrimidin (Referenzbeispiel 2) wie unter Beispiel 1 beschrieben.
Schmp. 210-215°C dec,( als Hydrochlorid aus Aceton).

**Beispiel 3:** 2-{2-[2(N,N-Dimethylamino)ethyl]oxy-phenyl}-4-phenyl-pyrimidin

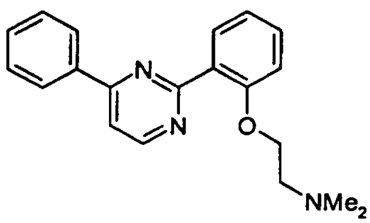

Die Darstellung erfolgte in Analogie zu den Beispielen 1 und 2.

**Beispiel 4:** 2-{2-[2(N,N-Dimethylamino)ethyl]oxy-phenyl}-4-phenyl-1,3,5-triazin

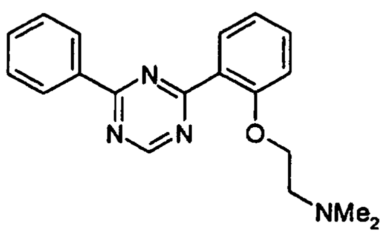

a) 2-(2-Dimethylamino-ethoxy)-benzamid

Zu einer Lösung von 2-Hydroxy-benzamide (10 g) und Kaliumcarbonat (35.4 g) in Dioxan (50 ml) and Wasser (4.5 ml) wurde N,N-dimethyl-2-chloroethylaminhydrochlorid (6.5 g) zugesetzt. Die Mischung wurde für 3 Stunden auf 85°C erhitzt. Das Lösemittel wurde entfernt, der erhaltene Rückstand in 5% HCl gegeben und mit Diethylether extrahiert. Die wässrige Phase wurde mittels $NaHCO_3$ auf pH 9 eingestellt und anschließend mit Ethylacetat extrahiert. Die organische Phase wurde getrocknet und die Titelverbindung (5.7g) nach Abdampfen des Lösemittels als weißer Feststoff erhalten.

b) 2-(2-Dimethylamino-ethoxy)-N,N-dimethylamino-methylene benzamid

Eine Mischung von 2-(2-Dimethylamino-ethoxy)-benzamid (3 g) und N,N-Dimethylformamide-dimethylacetal (4.2 ml) wurde für 3 h bei 110-120°C gerührt. Das entstehende Methanol wurde zunächst bei Normaldruck, später im Hochvakuum abdestilliert. Die Zielverbindung (helles Öl) wurde ohne weitere Reinigung weiter umgesetzt.

c) 5-{2-[2(N,N-Dimethylamino)ethyl]oxy-phenyl}-3-phenyl-1,2,4-triazin

Zu einer Lösung von 2-(2-N,N-Dimethylamino-ethoxy)-N,N-dimethylaminomethylenbenzamid (4 g) in wasserfreiem THF unter Stickstoffatmosphäre, wurde Benzamidine-hydrochtorid (2.34 g) und t-BuOK (1.68 g) zugesetzt. Die Mischung wurde für 3 h bei 55°C gerührt, das Lösemittel entfernt und das Rohprodukt mittels Säulenchromatographie auf neutralem Alumina gereinigt (Laufmittel: n-Hexan-Ethylacetat 1-1), was zur Zielverbindung in Form eines Öls führt; dieses wurde in Ethylacetat gelöst und mit der adaequaten Menge Oxalsäure versetzt. Das so erhaltene Oxalat der Zielverbindung wurde als weißer Feststoff erhalten.
Ausbeute: 0.2 g; Schmp. 126-129°C dec.

**Beispiel 5:** 5-{2-[2(N,N-Dimethylamino)ethyl]oxy-phenyl}-3-phenyl-1,2,4-triazin

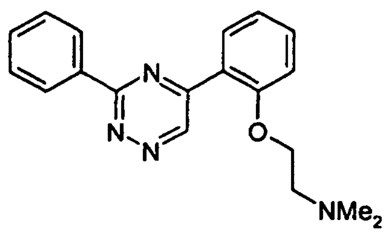

a) 1-[2-(2-dimethylamino-ethoxy)-phenyl]-ethanon

Die Darstellung erfolgte ausgehend von 1-(2-Hydroxyphenyl)-ethanon in Analogie zu Beispiel 4, Stufe a.

b) [2-(2-Dimethylamino-ethoxy)-phenyl]-oxo-acetaldehyd

Zu einer Lösung von 1-[2-(2-Dimethylamino-ethoxy)-phenyl]-ethanone (4.35 g) in DMSO (8.8 ml) wurde 48% HBr (1.8 ml) zugesetzt. Diese Mischung wurde bei 80°C für 6 h unter Stickstoffeinleitung in die Lösung gerührt. Anschließend wurde die Mischung in Methylenechlorid (50ml) gegeben und über $MgSO_4$ getrocknet. Die Mischung wird mit festem $NaHCO_3$ bis auf pH 7 neutralisiert, gefiltert und bei niedrigem Druck im Hochvakuum eingedampft. Die Zielverbindung wurde ohne weitere Reinigung verwendet.

c) 5-{2-[2(N,N-Dimethylamino)ethyl]oxy-phenyl}-3-phenyl-1,2,4-triazin

Zu einer Lösung von Benzocarboximidinsäure-hydrazid (0.9 g) in MeOH (10 ml) wurde bei 5°C langsam eine Lösung von [2-(2-Dimethylamino-ethoxy)-phenyl]-oxoacetaldehyd in MeOH (1.4 g) zugegeben. Die Mischung wurde bei 5°C für 6 h gerührt. Das Lösemittel wurde im Vakuum entfernt und das Rohprodukt durch Flash-Chromatographie an Kieselgel (Laufmittel: $CH_2Cl_2$-MeOH-$NH_4OH$ 95-5-0.5) geeinigt um die Zielverbindung als braunes Öl zu liefern. Diese wurde in Ethylacetat gelöst und mit einer adäquaten Menge Oxalsäure versetzt, um zum Oxalat der Titelverbindung in Form eines hellgelben Feststoffs zu führen.
Ausbeute: 0.240 g; Schmp. 167-170°C;

**Beispiel 6:** 3-{2-[2(N,N-Dimethylamino)ethyl]oxy-phenyl}-5-phenyl-1,2,4-triazin

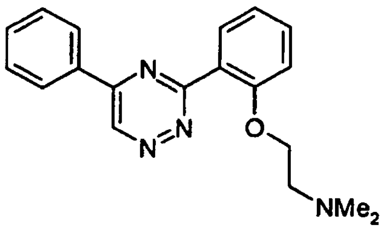

a) 2-(2-Dimethylamino-ethoxy)-benzonitril

Die Darstellung erfolgte ausgehend von 2-Hydroxybenzonitril in Analogie zu Beispiel 4, Stufe a.

b) 2-(2-Dimethylamino-ethoxy)-thiobenzamid

Eine Mischung von Trimethylsilylsulfid (5 g) und NaOMe (1.5 g) wurde tropfenweise zu einer gerührten Lösung von 2-(2-Dimethylamino-ethoxy)-benzonitril (2.66 g) wasserfreiem 1,3-Dimethyl-2-imidazolidinon gegeben. Die Mischung wurde bei Raumtemperatur 24 h gerührt, anschließend wurde sie mit Ethylacetat extrahiert und die organische Phase mit Wasser gewaschen, $MgSO_4$ getrocknet und eingedampft.
Das Rohprodukt wurde ohne weitere Reinigung verwendet.

c) 2-(2-Dimethylamino-ethoxy)-benzocarboximidic acid hydrazid

Zu einer Lösung von 2-(2-Dimethylamino-ethoxy)-thiobenzamid (9.4 g) in MeOH (50 ml) wurde eine 85%-ige wässrige Lösung von Hydrazine-hydrat (2.45 ml) langsam zugegeben. Die Mischung wurde bei Raumtemperatur für 4 h gerührt, das Lösemittel dann entfernt und das Rohprodukt durch Flash-Chromatographie an Kieselgel gereinigt (Laufmittel: $CH_2Cl_2$-MeOH-$NH_4OH$ 80-20-2), um die Zielverbindung (1.2 g) als braunes Öl zu liefern.

d) 3-{2-[2(N,N-Dimethylamino)ethyl]oxy-phenyl}-5-phenyl-1,2,4-triazin

Zu einer Lösung von 2-(N,N-Dimethylamino-ethoxy)-benzocarboximidinsäurehydrazid (1.2 g) in MeOH (10 ml) wird bei 5°C langsam eine Lösung von Phenylglyoxal-hydrat (0.82 g) in MeOH (8 ml) gegeben. Die Mischung wurde bei 5°C für 2 h gerührt, anschließend das Lösemittel im Vakuum entfernt und das erhaltene Rohprodukt durch Flash-Chromatography an Kieselgel (Laufmittel: $CH_2Cl_2$-MeOH-$NH_4OH$ 90-10-1 )gereinigt, um die Zielerbindung als gelbes Öl zu liefern. Dieses wurde in Ethylacetat gelösz und mit einer adäquaten Menge Oxalisäure versetzt, um das Oxalat der Zielverbindung als hellgelben Feststoff zu liefern.
Ausbeute: 0.260 g; Schmp. 175-180°C dec.

**Referenzbeispiel 3:** 5-(2-Methoxyphenyl)-2-phenyl-3H-pyrimidin-4-on

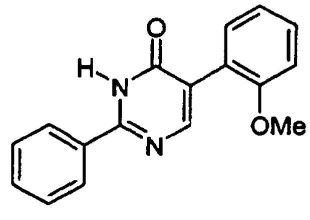

In eine Mischung von 2-Methoxy-phenylessigsäureethylester (11 g), Ethylformiat (8.2 ml) und Natrium (1.5

g) wurde tropfenweise EtOH (5 ml) bei 0 °C zugegeben und die erhaltene Mischung für 2 Tage bei Raumtemperatur gerührt. Wasser wurde zugegeben, die organische Phase wurde abgetrennt, die wässrige Phase nochmals mit Diethylether gewaschen, angesäuert und dreimal mit Ethylacetat extrahiert. Die vereinten Extrakte werden mit Wasser gewaschen, getrocknet und eingedampft, um zu 2 g Ethyl-2-(2-methoxy-phenyl)-3-oxo-propionat zu führen. Diese Verbindung (2 g) wurde in Wasser (50 ml) gelöst, $Na_2CO_3$ (0.95 g) wurde zugegeben und in diese Mischung eine Lösung von Benzamidine (1.1 g) in Wasser getropft. Die erhaltene Mischung wurde unter Rühren für 10 h auf 60 °C erwärmt, angesäuert und dreimal mit Methylenechlorid extrahiert. Die verinten Extrakte wurden mit 8%-iger wässriger $NaHCO_3$-Lösung und Wasser gewaschen, getrocknet und eingedampft. Der feste Rückstand wurde aus Diethylether umkristallisiert, um die Titelverbindung als weißen Feststoff zu liefern (0.4 g, Schp. 245-7 °C).

**Referenzbeispiel 4:** 5-(2-Methoxyphenyl)-3-methyl-2-phenyl-pyrimidin-4-on

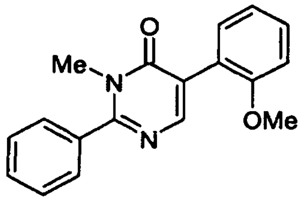

Eine Mischung von 5-(2-Methoxyphenyl)-2-phenyl-3H-pyrimidin-4-on (0.4 g, Beispiel 9), Methyliodid (0.134 ml), $K_2CO_3$ (0.398 g) und Tetrabutylammoniumbromide (0.048 g) in Toluol (60 ml) and Wasser (10 ml) wurde für 2 h zum Rückfluß erhitzt und eingedampft. Der Rückstand wurde in Diethylether aufgenommen und unlösliches Material abfiltriert. Die etherische Lösung wurde eingedampft und der Rückstand durch Flash-Chromatographie an Kieselgel (Laufmittel: n-Hexan-Ethylacetate 6-4) gereinigt, um die Titelverbindung in Form eines weißen Feststoffs zu liefern (0.22 g).
Schp. 119-120 °C.

Überraschenderweise wurde gefunden, daß die erfindungsgemäßen Verbindungen eine Affinität zu oder Aktivität an verschiedenen Rezeptortypen zeigen und eine neuroprotektive Wirkung aufweisen.

*In vitro* und *in vivo* Versuche haben gezeigt, daß die im Gehirn infolge von Hypoglykämie, Hypoxie, Anoxie, globale und fokale Ischämie, Schädel-Hirn-Trauma, Hirn-Ödem und Hirn-Druck auftretenden Zellschäden und Funktionsausfälle z. T. auf einer erhöhten synaptischen Aktivität und somit vermehrter Transmitterfreisetzung beruhen. Neben Glutamat sind Histamin und Serotonin als Neurotransmitter von besonderer Bedeutung. Darüberhinaus werden die Konzentrationen von insbesondere Calcium und Natrium Ionen verändert.

Es ist bekannt, daß nach systemischer Applikation von Glutamat Neuronen im Gehirn von Mäusen zerstört werden (S.M. Rothman und T.W. Olney, Trends in Neurosciences 10 (1987) 299). Dieser Befund läßt unter anderem den Schluß zu, daß Glutamat eine Rolle bei neurodegenerativen Erkrankungen spielt (R.Schwarcz und B. Meldrum, The Lancet 11 (1985) 140). Weiterhin sind Substanzen wie z.B. Quisqualinsäure, Kaininsäure, Ibotensäure, Glutaminsäure, N-Methyl-D-asparaginsäure (NMDA) und α-Amino-3-hydroxy-5-methyl-4-isooxazol-propionsäure (AMPA) als exogene bzw. endogene Neurotoxine bekannt. Gehirnläsionen, die mit solchen Substanzen induziert werden können, sind vergleichbar mit jenen, welche mit Zusammenhang mit Epilepsie und anderen neurodegenerativen Erkrankungen - wie z.B. Morbus Huntington und Morbus Alzheimer - auftreten. Substanzen und Ionen, welche die Aktivität der Glutamat-Rezeptoren und des mit diesem Rezeptor verbundenen Ionenkanals hemmen - wie z.B. kompetitive und nichtkompetitive Antagonisten exzitatorischer Aminosäuren - schützen Gehirnzellen vor hypoxischen bzw. ischämischen Schäden. Diese Befunde zeigen, daß die Glutamat-Rezeptoren eine wichtige Rolle bei der Vermittlung des ischämischen Schadens spielen.

Der Nachweis der Wirkung am AMPA Rezeptor wurde mittels Elektrophysiologie an neuronalen Zellen (Patch-Clamp-Methode) geführt (M. L. Mayer, L. Vyklicky and G. L. Westbrook, J. Physiol. 415 (1989) 329-350).
Die Testung erfolgte bei einer Testkonzentration von 100 μM.

Tabelle 1:

| Hemmung des Kainat-induzierten Signals am AMPA-Rezeptor | |
|---|---|
| Beispiel | AMPA Inh. [%] |
| 2 | 96 |

Tabelle 1: (fortgesetzt)

| Hemmung des Kainat-induzierten Signals am AMPA-Rezeptor | |
|---|---|
| Beispiel | AMPA Inh. [%] |
| 4 | 66 |
| 5 | 98 |
| 6 | 95 |

Der Nachweis der Affinität zur "$Na^+$ Kanal site 2"-Bindungsstelle wurde wie von G.B. Brown (J. Neurosci. 6 (1986) 2064) beschrieben geführt. Die Testung erfolgte typischerweise bei einer Testkonzentration von 10µM.

Tabelle 2:

| Beispiel | Ki [µm] |
|---|---|
| 2 | 4 |
| 4 | 2,8 |
| 5 | 2,4 |

Die oben beschriebenen Ergebnisse zeigen, daß die Verbindungen der allgemeinen Formel (I) bei neurodegenerativen Erkrankungen sowie Gehirnischämie verschiedener Genese eingesetzt werden können. Hierunter fallen beispielsweise: Status epileptikus, Hypoglykämie, Hypoxie, Anoxie, Gehirntrauma, Gehirnoedem, amyotrope laterale Sklerose, Huntington's Disease, Morbus Alzheimer, Hypotonie, Herzinfarkt, Hirndruck (erhöhter intrakranialer Druck), ischämischer und hämorrhagischer Stroke, globale cerebrale Ischämie bei Herzstillstand, Diabetische Polyneuropathie, Tinitus, perinatale Asphyxie, Psychose, Schizophrenie, Depression und Morbus Parkinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parenteral - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.
Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Ferner können die Verbindungen der allgemeinen Formel I bzw. deren Säureadditionssalze auch mit andersartigen Wirkstoffen kombiniert werden.

Die nachfolgenden Beispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstarke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |

(fortgesetzt)

| B) | Tabletten | pro Tablette |
|---|---|---|
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

**Patentansprüche**

**1.** Verbindungen der allgemeinen Formel (I)

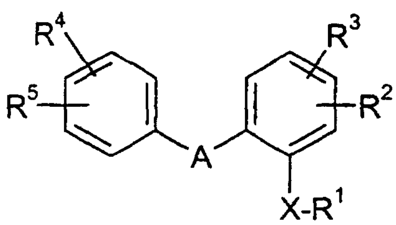

(I)

worin

A ein 6-gliedriger gesättigter oder ungesättigter Heterocyclus, der als Heteroatome 1, 2 oder 3 Stickstoffatome enthalten kann und gegebenenfalls ein- oder mehrfach durch =O oder $C_1$-$C_4$-Alkyl substituiert ist;

X Sauerstoff;

$R^1$ $C_1$-$C_4$-Alkyl, das durch $-NR^6R^7$ oder durch ein N-Oxid der Formel $-NOR^6R^7$ substituiert ist;

$R^2$ und $R^3$ Wasserstoff;

$R^4$ und $R^5$ Wasserstoff;

$R^6$ Wasserstoff oder $C_1$-$C_4$-Alkyl;

$R^7$ Wasserstoff oder $C_1$-$C_4$-Alkyl, oder

$R^6$ und $R^7$ bilden zusammen mit dem Stickstoffatom einen gesättigten oder ungesättigten Heterocyclus ausgewählt aus der Gruppe Pyrrol, Pyrrolin, Pyrrolidin, Piperidin, Piperazin, Morpholin, Thiomorpholin,

Imidazol, Imidazolin, Imidazolidin, Pyrazol, Pyrazolin, Pyrazolidin, wobei die genannten Heterocyclen gegebenenfalls durch Methyl, Ethyl, Propyl oder Benzyl substituiert sein können, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1, worin

| | |
|---|---|
| A | ein 6-gliedriger Heterocyclus, ausgewählt aus der Gruppe Pyridin, Pyridazin, Pyrimidin, Pyrazin oder Triazin, der gegebenenfalls durch =O oder $C_1$-$C_4$-Alkyl substituiert sein kann; |
| X | Sauerstoff; |
| $R^1$ | Methyl, Ethyl oder Propyl, die jeweils durch $-NR^6R^7$ oder durch ein N-Oxid der Formel $-NOR^6R^7$ substituiert sind; |
| $R^2$ und $R^3$ | Wasserstoff; |
| $R^4$ und $R^5$ | Wasserstoff; |
| R6 | Wasserstoff, Methyl, Ethyl oder Propyl; |
| $R^7$ | Wasserstoff, Methyl, Ethyl oder Propyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze. |

**3.** Verbindungen der allgemeinen Formel (I) gemäß einem der Ansprüche 1 oder 2 worin die Gruppe

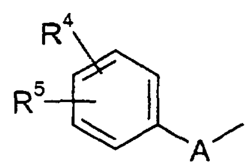

für einen der folgenden Reste stehen kann

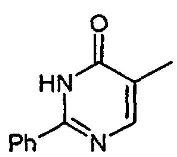 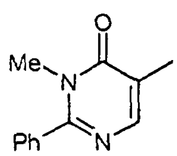 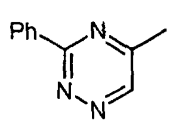 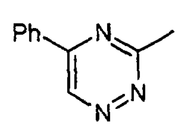

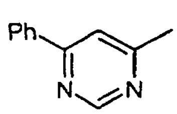 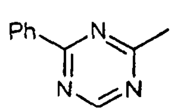 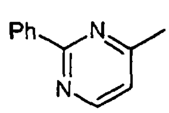 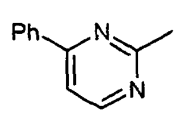 ;

| | |
|---|---|
| X | Sauerstoff; |
| $R^1$ | Ethyl oder Propyl, die jeweils durch $-NR^6R^7$ substituiert sind; |
| $R^2$ und $R^3$ | Wasserstoff; |
| $R^6$ | Methyl, Ethyl oder Propyl; |

$R^7$ Methyl, Ethyl oder Propyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**4.** Verbindungen der allgemeinen Formel (I) gemäß einem der Ansprüche 1 bis 3 worin die Gruppe

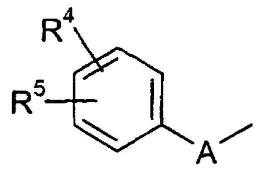

für einen der folgenden Reste stehen kann

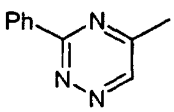 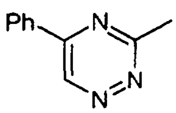 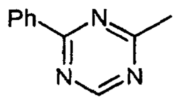 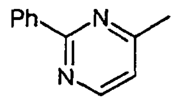 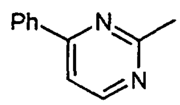 ;

X Sauerstoff;

$R^1$ $-CH_2-CH_2-NR^6R^7$;

$R^2$ und $R^3$ Wasserstoff;

$R^6$ Methyl;

$R^7$ Methyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**5.** Verwendung einer Verbindung der allgemeinen Formel (I) gemäß Anspruch 1 bis 4 zur Herstellung eines Arzneimittels.

**6.** Verwendung einer Verbindung der allgemeinen Formel (I) gemäß Anspruch 1 bis 4 zur Herstellung eines Arzneimittels zur Behandlung von neurodegenerativen Erkrankungen sowie Gehirnischämie verschiedener Genese.

**7.** Pharmazeutische Zubereitungen, enthaltend als Wirkstoff eine oder mehrere Verbindungen der allgemeinen Formel (I) gemäß den Ansprüchen 1 bis 4 oder deren physiologisch verträgliche Säureadditionssalze in Kombination mit üblichen Hilfs- und/oder Trägerstoffen.

**8.** Verfahren zur Herstellung von Verbindungen der allgemeinen Formel (I)

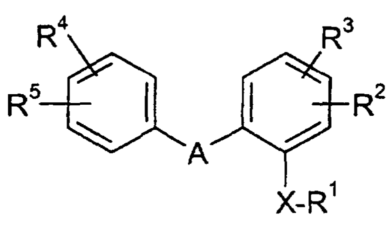

(I)

worin A, X und die Reste $R^1$, $R^2$, $R^3$, $R^4$ und $R^5$ die in den Ansprüchen 1 bis 4 genannte Bedeutung aufweisen, **dadurch gekennzeichnet, daß** eine Verbindung der allgemeinen Formel (II)

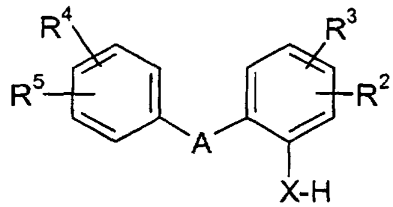

(II)

worin A, X und die Reste $R^2$, $R^3$, $R^4$ und $R^5$ die in den Ansprüchen 1 bis 4 genannte Bedeutung aufweisen, unter basischen Bedingungen mit Elektrophilen der allgemeinen Formel

$$L\text{-}R^1,$$

wobei L eine Abgangsgruppe wie beispielsweise Chlor, Brom, Iod, Methansulfonyl, Trifluormethansulfonyl oder p-Toluolsulfonyl darstellt und $R^1$ die in den Ansprüchen 1 bis 4 angegebene Bedeutung aufweist, umgesetzt wird.

## Claims

**1.** Compounds of general formula (I)

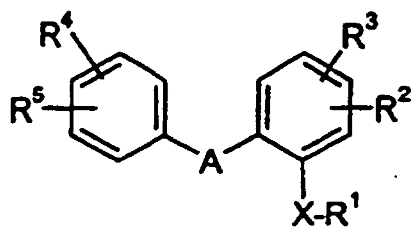

(I)

wherein

| | |
|---|---|
| A | denotes a 6-membered saturated or unsaturated heterocycle which may contain 1, 2 or 3 nitrogen atoms as heteroatoms and is optionally mono- or polysubstituted by =0 or $C_{1-4}$-alkyl; |
| X | denotes oxygen; |
| $R^1$ | denotes $C_{1-4}$-alkyl which is substituted by $-NR^6R^7$ or by an N-oxide of the formula $-NOR^6R^7$; |
| $R^2$ and $R^3$ | denote hydrogen; |
| $R^4$ and $R^5$ | denote hydrogen; |
| $R^6$ | denotes hydrogen or $C_{1-4}$-alkyl; |
| $R^7$ | denotes hydrogen or $C_{1-4}$-alkyl; or |
| $R^6$ and $R^7$ | together with the nitrogen atom form a saturated or unsaturated heterocycle selected from among pyrrole, pyrroline, pyrrolidine, piperidine, piperazine, morpholine, thiomorpholine, imidazole, imidazoline, imidazolidine, pyrazole, pyrazoline, pyrazolidine, the said heterocycles optionally being substituted by methyl, ethyl, propyl or benzyl, |

optionally in the form of the racemates, the enantiomers, in the form of the diastereomers and mixtures thereof,

and optionally the pharmacologically acceptable acid addition salts thereof.

**2.** Compounds of general formula (I) according to claim 1, wherein

A denotes a 6-membered heterocycle selected from among pyridine, pyrimidine, pyrazine or triazine, which may optionally be substituted by =0 or $C_{1-4}$-alkyl;

X denotes oxygen;

$R^1$ denotes methyl, ethyl or propyl, each of which is substituted by. $-NR^6R^7$ or by an N-oxide of the formula $-NOR^6R^7$;

$R^2$ and $R^3$ denote hydrogen;

$R^4$ and $R^5$ denote hydrogen;

$R^6$ denotes hydrogen, methyl, ethyl or propyl;

$R^7$ denotes hydrogen, methyl, ethyl or propyl,

optionally in the form of the racemates, the enantiomers, in the form of the diastereomers and mixtures thereof, and optionally the pharmacologically acceptable acid addition salts thereof.

**3.** Compounds of general formula (I) according to one of claims 1 and 2, wherein the group

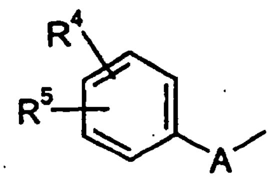

may denote one of the following groups

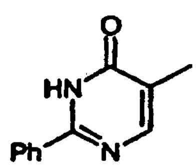
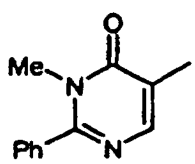
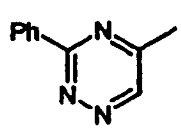
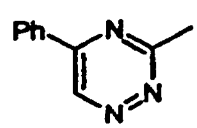

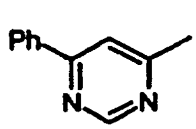
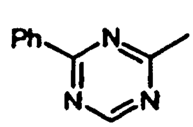
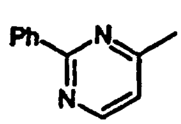
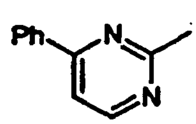

X denotes oxygen;

$R^1$ denotes ethyl or propyl, each of which is substituted by $-NR^6R^7$;

$R^2$ and $R^3$ denote hydrogen;

$R^6$ denotes methyl, ethyl or propyl;

$R^7$ denotes methyl, ethyl or propyl,

optionally in the form of the racemates, the enantiomers, in the form of the diastereomers and mixtures thereof, and optionally the pharmacologically acceptable acid addition salts thereof.

**4.** Compounds of general formula (I) according to one of claims 1 to 3, wherein the group

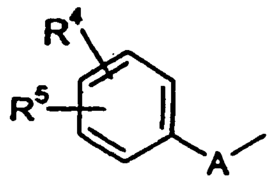

may denote one of the following groups

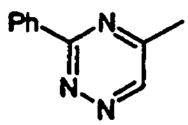 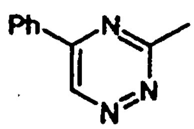 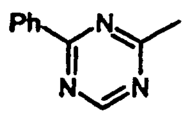 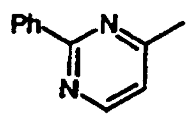 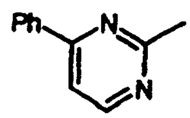

X denotes oxygen;

$R^1$ denotes $-CH_2-CH_2-NR^6R^7$;

$R^2$ and $R^3$ denote hydrogen;

$R^6$ denotes methyl;

$R^7$ denotes methyl,

optionally in the form of the racemates, the enantiomers, in the form of the diastereomers and mixtures thereof, and optionally the pharmacologically acceptable acid addition salts thereof.

**5.** Use of a compound of general formula (I) according to claims 1 to 4 for preparing a pharmaceutical composition.

**6.** Use of a compound of general formula (I) according to claims 1 to 4 for preparing a pharmaceutical composition for the treatment of neurodegenerative diseases and cerebral ischaemia of various origins.

**7.** Pharmaceutical preparations containing as active substance one or more compounds of general formla (I) according to claims 1 to 4 or the physiologically acceptable acid addition salts thereof combined with conventional excipients and/or carriers.

**8.** Process for preparing compounds of general formula (I)

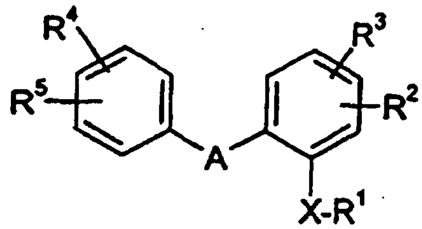

wherein A, X and the groups $R^1$, $R^2$, $R^3$, $R^4$ and $R^5$ have the meanings given in claims 1 to 4, **characterised in that** a compound of general formula (II)

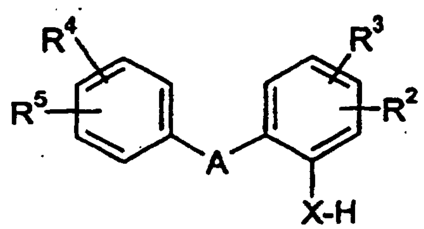

wherein A, X and the groups $R^2$, $R^3$, $R^4$ and $R^5$ have the meanings given in claims 1 to 4, is reacted under basic conditions with electrophils of general formula

$$L\text{-}R^1,$$

wherein L is a leaving group such as, for example, chlorine, bromine, iodine, methanesulphonyl, trifluoromethanesulphonyl or p-toluenesulphonyl and $R^1$ has the meanings given in claims 1 to 4.

## Revendications

**1.** Composés de formule générale (I)

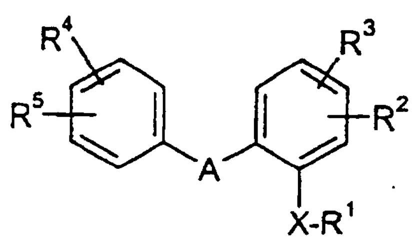

(I)

où

A représente un hétérocycle à 6 chaînons saturé ou insaturé, qui peut contenir comme hétéroatomes 1, 2 ou 3 atomes d'azote et qui est éventuellement substitué une ou plusieurs fois par =O ou $C_1$-$C_4$-alkyle ;

X représente l'oxygène ;

$R^1$ représente $C_1$-$C_4$-alkyle, qui est substitué par $-NR^6R^7$ ou par un N-oxyde de formule $-NOR^6R^7$ ;

$R^2$ et $R^3$ représentent l'hydrogène ;

$R^4$ et $R^5$ représentent l'hydrogène ;

$R^6$ représente l'hydrogène ou $C_1$-$C_4$-alkyle ;

$R^7$ représente l'hydrogène ou $C_1$-$C_4$-alkyle,

ou
$R^6$ et $R^7$ forment avec l'atome d'azote un hétérocycle saturé ou insaturé choisi dans le groupe de pyrrole, pyrroline, pyrrolidine, pipéridine, pipérazine, morpholine, thiomorpholine, imidazole, imidazoline, imidazolidine, pyrazole, pyrazoline, pyrazolidine, où les hétérocycles cités peuvent éventuellement être substitués par méthyle, éthyle, propyle ou benzyle, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**2.** Composés de formule générale (I) selon la revendication 1, où

A représente un hétérocycle à 6 chaînons choisi dans le groupe de pyridine, pyridazine, pyrimidine, pyrazine et triazine, qui peut éventuellement être substitué par =O ou $C_1$-$C_4$-alkyle ;

X représente l'oxygène ;

$R^1$ représente méthyle, éthyle ou propyle, qui sont substitués dans chaque cas par - $NR^6R^7$ ou par un N-oxyde de formule $-NOR^6R^7$ ;

$R^2$ et $R^3$ représentent l' hydrogène ;

$R^4$ et $R^5$ représentent l'hydrogène ;

$R^6$ représente l'hydrogène, méthyle, éthyle ou propyle ;

$R^7$ représente l'hydrogène, méthyle, éthyle ou propyle ,

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**3.** Composés de formule générale (I) selon l'une des revendications 1 et 2 où le groupe

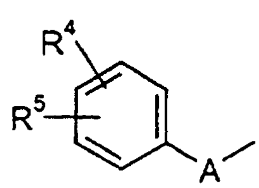

peut représenter l'un des groupements suivants

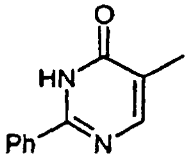
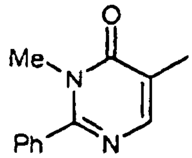
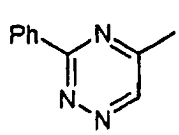
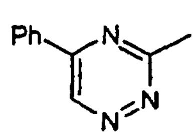

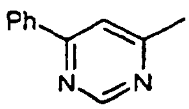 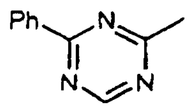 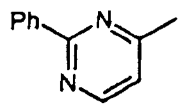 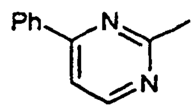 ;

X représente l'oxygène ;

$R^1$ représente éthyle ou propyle, qui sont substitués dans chaque cas par $-NR^6R^7$ ;

$R^2$ et $R^3$ représentent l'hydrogène ;

$R^6$ représente méthyle, éthyle ou propyle ;

$R^7$ représente méthyle, éthyle ou propyle ,

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**4.** Composés de formule générale (I) selon l'une des revendications 1 à 3 où le groupe

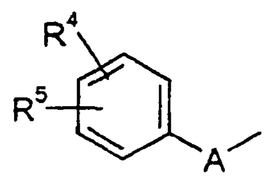

peut représenter l'un des groupements suivants

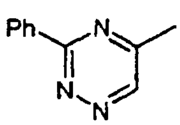 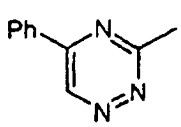 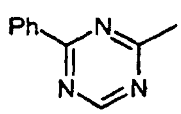 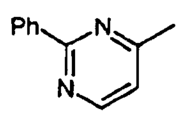 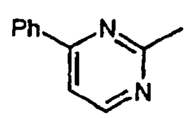 ;

X représente l'oxygène ;

$R^1$ représente $-CH_2-CH_2-NR^6R^7$ ;

$R^2$ et $R^3$ représentent l'hydrogène ;

$R^6$ représente méthyle ;

$R^7$ représente méthyle, éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**5.** Utilisation d'un composé de formule générale (I) selon les revendications 1 à 4 pour la production d'un médicament.

**6.** Utilisation d'un composé de formule générale (I) selon les revendications 1 à 4 pour la production d'un médicament pour le traitement des maladies neurodégénératives ainsi que de l'ischémie cérébrale de genèse variable.

**7.** Préparations pharmaceutiques contenant comme principe actif un ou plusieurs composés de formule générale (I) selon les revendications 1 à 4 ou leurs sels d'addition d'acide physiologiquement acceptables en combinaison avec des adjuvants et/ou supports courants.

**8.** Procédé de préparation de composés de formule générale (I)

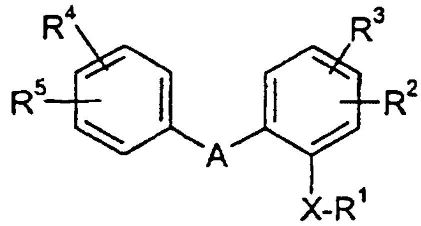

(I)

où A, X et les groupements $R^1$, $R^2$, $R^3$, $R^4$ et $R^5$ présentent la signification citée dans les revendications 1 à 4 **caractérisé en ce qu'**un composé de formule générale (II)

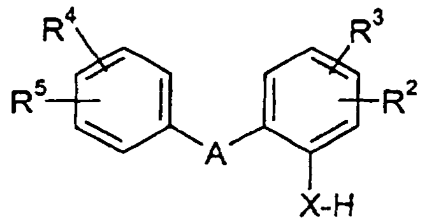

(II)

où A, X et les groupements $R^2$, $R^3$, $R^4$ et $R^5$ présentent la signification citée dans les revendications 1 à 4 est mis à réagir dans des conditions basiques avec des électrophiles de formule générale

$$L\text{-}R^1$$

où L représente un groupe partant comme, par exemple, le chlore, le brome, l'iode, méthanesulfonyle, trifluorométhanesulfonyle ou p-toluènesulfonyle et $R^1$ présente la signification indiquée dans les revendications 1 à 4.